# FASCICULE DE BREVET EUROPEEN

(11) **EP 0 836 452 B1**
(45) Date de publication et mention de la délivrance du brevet: **10.12.2003**
(21) Numéro de dépôt: 97921877.3
(22) Date de dépôt: 24.04.1997
(51) Int. Cl.: A61F 2/06

(54) **PROTHESE VASCULAIRE TRANSCUTANEE CHIRURGICALEMENT ANASTOMOSABLE ET ENSEMBLE COMPRENANT UNE TELLE PROTHESE**
CHIRURGISCHE, ZUR ANASTOMOSE FÄHIGE, TRANSDERMALE GEFÄSSPROTHESE SOWIE EIN MIT DIESER PROTHESE AUSGESTALTETER BAUSATZ
SURGICALLY ANASTOMOSABLE TRANSCUTANEOUS VASCULAR PROSTHESIS AND SET COMPRISING SAME

(30) Priorité: 02.05.1996 FR 9605505; 08.04.1997 FR 9704288
(43) Date de publication de la demande: 22.04.1998
(73) Titulaire: B. BRAUN CELSA (société anonyme), 86360 Chasseneuil du Poitou (FR)
(72) Inventeur: ANIDJAR, Samy, F-75009 Paris (FR); CHEVILLON, Gérard, F-92120 Montrouge (FR)
(74) Mandataire: Lerner, François
(86) Numéro de dépôt international: FR9700734
(87) Numéro de publication internationale: WO97041804

(56) Documents cités:
- EP-A- 0 556 850
- EP-A- 0 646 365
- WO-A-88/06026
- WO-A-95/21592
- WO-A-96/24306
- US-A- 4 562 596
- DATABASE WPI Section PQ, Week 8932 Derwent Publications Ltd., London, GB; Class P32, AN 89-233554 XP002035929 & SU 1 457 921 A (KHARK EMERG SURGERY (KHMO=)) , 15 Février 1987

## Description

L'invention vise à améliorer les conditions actuelles de "traitement" de zones endommagées de certains conduits anatomiques.

Il s'agit en particulier d'améliorer les conditions de circulation du sang dans une zone vasculaire endommagée, tout particulièrement si le(les) vaisseau(x) présente(nt) un ou plusieurs anévrismes.

Et plus particulièrement encore, il s'agit d'améliorer les conditions actuelles de traitement des anévrismes débordant sur plusieurs vaisseaux communicants, tout particulièrement pour des anévrismes d'aorte débordant sur un ou plusieurs des vaisseaux iliaques, voire même sur l'artère hypogastrique.

Aujourd'hui, pour pallier certaines dégénérescences ou certains endommagements de conduits anatomiques, il est connu d'utiliser des prothèses tubulaires implantables par voie intraluminale percutanée (technique de SELDINGER en particulier).

Ces prothèses endoscopiques (ou endoprothèses) qui ont pour objet de former au moins localement un substitut du conduit, comprennent souvent :
- un manchon ou gaine sensiblement non "autoportant(e)", et
- une armature (ou stent) tubulaire essentiellement coaxiale à la gaine, cette armature étant adaptée pour être radialement resserrée ou déployée.

L'armature sert alors de support au manchon (qui n'a donc habituellement pas de "tenue" intrinsèque et s'affaisse s'il n'est pas maintenu) et permet l'implantation endoluminale percutanée de la prothèse, le manchon s'adaptant à la forme de son armature, notamment pour former un tube permettant de canaliser un fluide corporel, lorsque l'armature est radialement déployée.

Des exemples de telles prothèses formant localement un substitut de conduit sont décrits notamment dans US-A- 5 282 824. Dans WO-A-95/21592, il est même prévu d'adjoindre à une telle prothèse bifurquée, une prothèse tubulaire d'allonge facilitant la pose de la prothèse bifurquée dont les deux branches secondaires issues de la bifurcation peuvent être alors courtes, tout en permettant d'adapter à volonté la longueur de l'allonge.

Mais ces implants vasculaires introduits par voie endoluminale percutanée peuvent être déconseillés dans certains cas cliniques, tout particulièrement lorsque l'(les) anévrime(s) est(sont) trop étendu(s) et, partant de l'aorte, se développe(nt) sur plusieurs vaisseaux, au-delà de la bifurcation iliaque (iliaques externe et/ou interne), voire se prolonge(nt) jusqu'à l'artère hypogastrique.

Une solution peut alors consister à implanter chirurgicalement une prothèse vasculaire de substitution locale de vaisseau, de type "pontage". Mais ceci implique une intervention chirurgicale "lourde", pour découvrir toute(s) la(les) zone(s) vasculaire(s) endommagée(s), en général avec dénudation thoracique et/ou abdominale. Une telle prothèse est par exemple décrite dans WO-A-8806026 ou US-A-3 986 828.

Or, l'invention souhaite justement éviter cela, en se ralliant ainsi aux motifs qui ont conduit à l'adoption des implants endoluminaux percutanés.

Le document EP-A-0 646 365 décrit une prothèse vasculaire adaptée pour être implantée par voie endoluminale percutanée, comprenant :
- un manchon souple pour y canaliser du sang,
- et une armature tubulaire à laquelle est lié le manchon pour que la prothèse se présente comme un tube unique ou bifurqué, la prothèse comprenant en outre une portion terminale vasculairement anastomosable de manchon qui prolonge le manchon souple, cette portion terminale étant donc adaptée pour être anastomosée, en particulier par suture, à au moins un vaisseau ou substitut de vaisseau. Il est ainsi possible de réaliser un acte chirurgical de liaison (en particulier par suture) entre la portion terminale de manchon et le conduit anatomique considéré (vaisseau) ou un substitut de conduit, si celui-ci est trop endommagé à l'endroit où ce tronçon de prothèse a été mis en place.

Le document EP-A-0 556 850 décrit une prothèse analogue à celle du document EP-A-0 646 365.

Le document US-A-4 562 596 décrit une prothèse comportant un manchon souple et une armature, et ayant une partie terminale vasculairement anastomosable qui prolonge le manchon au-delà de l'armature.

Le document DATABASE WPI Section PQ, semaine 8962, Derwent Publication, Ltd, London, Classe P32, AN89-233554 XP002035929, décrit une prothèse analogue ayant un élément de fixation distant du manchon auquel il est lié par des tiges élargies et destiné à empêcher la migration de la prothèse.

Un problème que l'invention s'est attachée à résoudre concerne l'étanchéité vis-à-vis du sang entre la portion terminale anastomosable du manchon et le vaisseau sanguin (coupé) à travers lequel cette portion aura été engagée.

En effet, pour assurer l'anastomose entre cette portion terminale et le vaisseau (ou substitut) considéré, en aval de l'anévrisme, il a nécessairement fallu sectionner là le vaisseau. Pour la partie aval, l'anastomose assure une étanchéité appropriée entre ce vaisseau (ou substitut) et la prothèse. Reste le problème de la partie amont qui reçoit le flux sanguin.

La solution proposée dans l'invention consiste à adjoindre à la prothèse un moyen annulaire, tel qu'une bague, (étanche) d'appui à disposer autour de la portion terminale anastomosable de manchon pour obtenir à la fois une étanchéité vis-à-vis du sang entre ledit moyen annulaire et ce tronçon terminal, et un moyen de fixation étanche pour fixer ainsi, autour de ce moyen d'appui, le tronçon de vaisseau coupé à travers lequel est passée cette portion terminale anastomosable.

Le moyen de fixation pourra être un lien que le praticien viendra donc serrer autour de la partie amont du vaisseau considéré, plaquant ainsi cette partie contre la "bague" d'appui. Pour assurer l'étanchéité face au sang entre la "bague" et la portion terminale de manchon, la première pourra être engagée très étroitement autour de la seconde et/ou un moyen complémentaire d'étanchéité rapporté pourra être prévu, tel qu'un tampon de mousse ou de tissu à placer sous la "bague" ou encore un rabat souple en une fine matière imperméable au sang, fixée autour de la portion considérée du manchon (à l'écart de son extrémité libre à anastomoser) et pliée par dessus la "bague".

Dans le cas où l'affection vasculaire concerne un ou plusieurs anévrisme(s) "compliqué(s)", une telle prothèse (implantable par voie endoluminale percutanée) peut être utilisée pour le traitement d'un débordement d'anévrisme sur plusieurs vaisseaux ramifiés et communiquant entre eux, ce traitement s'opérant, une fois la prothèse implantée, par chirurgie vasculaire (avec anastomose) entre la portion terminale de manchon percutanée vasculairement anastomosable qui prolonge donc (ou est rapportée sur) ledit manchon souple de la prothèse et le vaisseau ou substitut de vaisseau considéré.

Pour un traitement anévrismal, la prothèse de l'invention sera avantageusement une branche prothétique d'une prothèse bifurquée comprenant, pour canaliser le sang, un tronçon tubulaire principal raccordé à des tronçons tubulaires secondaires dont l'un au moins sera adapté pour être pourvu de ladite branche prothétique à portion extrème libre dépourvue d'armature de soutien (portion anastomosable).

Selon un autre aspect, l'invention s'est par ailleurs attachée à définir les conditions de réalisation du manchon (qui peut être en une ou plusieurs parties) de l'une des prothèses définies ci-avant.

La solution proposée consiste avantageusement en ce que :
- la partie du manchon située essentiellement en regard du stent est radialement extensible et/ou n'occupe sa forme tubulaire radialement ouverte que sous l'action de ce stent qui lui sert d'armature (en la maintenant) et qui présente alors son deuxième diamètre,
- la portion terminale anastomosable de manchon est moins extensible radialement que l'autre portion et/ou occupe naturellement d'elle-même une forme tubulaire radialement ouverte.

En outre, les matières ou textures de constitution et/ou les épaisseurs desdites parties de manchon respectivement en regard de l'armature et anastomosable seront de préférence différentes.

Parmi ces caractéristiques, l'épaisseur et la tenue mécanique (autoportance) sont certainement les plus importantes.

En effet, on peut raisonnablement considérer que l'autoportance susmentionnée de la portion terminale anastomosable lui permet d'occuper naturellement (hors contrainte mécanique) une forme radialement ouverte (sensiblement circulaire en particulier), d'être (essentiellement) étanche au fluide corporel considéré (sang ; ceci étant au moins indirectement lié à l'épaisseur), sa rigidité "intrinsèque" relative permettant en outre au praticien de réaliser plus facilement les anastomoses. Si aujourd'hui l'épaisseur et/ou la matière ou texture de l'autre partie du manchon située essentiellement en regard de l'armature devenait par contre comparable à celle dudit tronçon anastomosable, alors il pourrait y avoir des difficultés pour introduire et/ou mettre en place l'implant par voie endoluminale transcutanée.

C'est pourquoi il apparaît préférable dans l'invention que la partie de manchon devant être déployée radialement par l'armature soit d'épaisseur inférieure à la portion terminale anastomosable qui se déploie d'elle-même (si elle a été préalablement déformée par resserrement radial).

Il n'en demeure pas moins que les matières ou textures de constitution de chacune des parties de manchon pourront jouer leur rôle, par exemple en améliorant l'étanchéité vis-à-vis du sang de la partie de manchon devant être déployée par l'armature, ou encore en permettant de rendre radialement extensible (de manière élastique ou non) cette même portion de manchon, en conservant par contre si nécessaire essentiellement non extensible radialement la portion terminale anastomosable.

Au sujet de ces caractéristiques d'extensibilité et de non déformabilité radiale (a priori en compression) sans effort notable, un critère important est la possible utilisation de manchons déjà commercialisés respectivement pour des implants vasculaires "transcutanés" endoluminaux et pour des implants vasculaires "chirurgicaux", type implant de "pontage", le raccordement bout à bout de ces deux types de manchons et leur utilisation respective déjà éprouvée permettant tant d'augmenter leur fiabilité que de diminuer leur coût de fabrication.

Ainsi, on va pouvoir combiner les avantages des implants vasculaires endoluminaux percutanés avec ceux des implants chirurgicaux de substitution vasculaire.

Concernant les "épaisseurs" de manchon auxquelles il a été fait référence plus haut, on notera qu'il s'agit d'épaisseurs totales, y compris une éventuelle épaisseur de gaufrage, d'ondulations, ou crée par tout autre moyen "antiplicature".

A noter également que l'invention s'est attachée à définir les proportions devant avantageusement être attribuées à ladite "portion terminale anastomosable" par rapport à la longueur totale du manchon de l'implant entre deux extrémités axiales opposées de celui-ci.

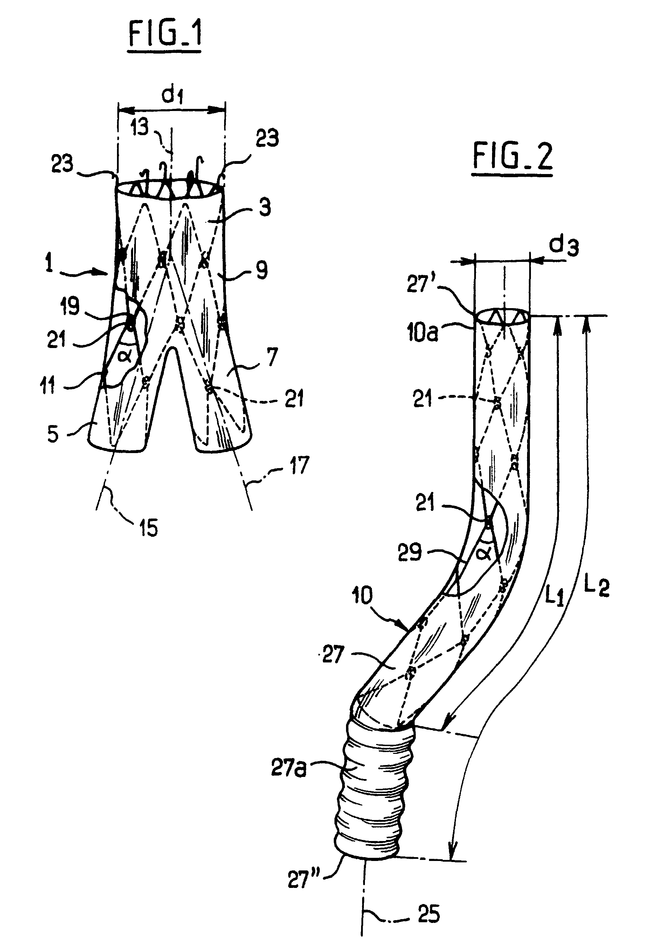

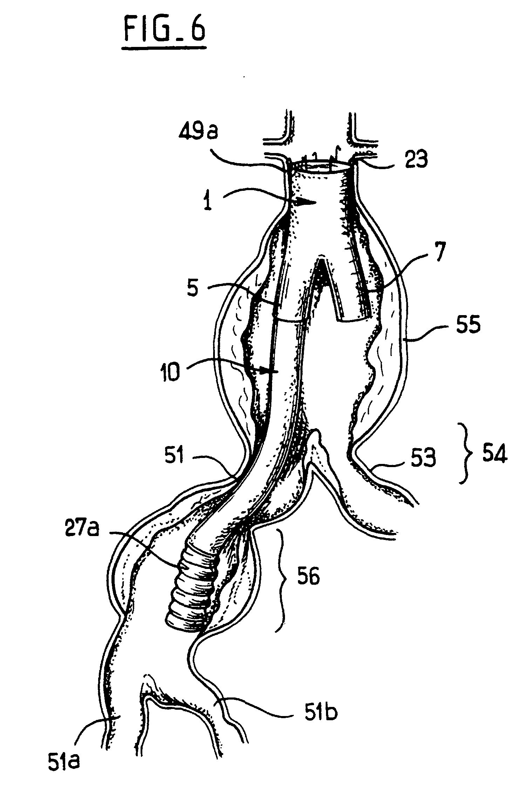

Dans un premier temps, il était apparu préférable de limiter cette longueur de "portion terminale" à une zone d'extrémité marginale (voir, par exemple, figures 2 ou 6 ci-après).

En particulier, on a d'abord supposé que l'implantation de l'ensemble de la prothèse par voie endoluminale percutanée imposerait que l'essentiel au moins de la longueur axiale de celle-ci soit réalisée sous la forme d'une "prothèse endoluminale traditionnelle", c'est-à-dire avec une armature du type précité soutenant un manchon pratiquement sans tenue mécanique intrinsèque (tissu ou équivalent très peu rigide).

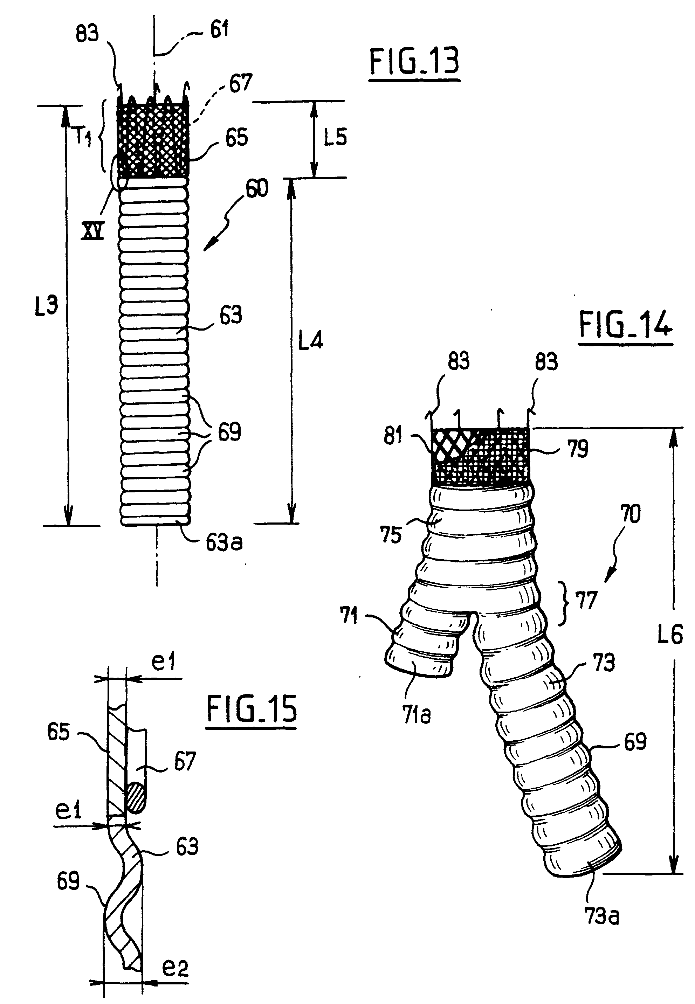

Or, il s'est avéré de façon surprenante qu'il peut être préférable que ce soit l'autre portion du manchon, dénommée ci-avant "terminale anastomosable", qui occupe l'essentiel de la longueur de ce manchon, à l'image de ce qui est illustré sur les figures 13 et 14 ci-après.

Ainsi, depuis donc une extrémité et sur l'essentiel de sa longueur, le manchon ne sera avantageusement soutenu par aucun stent ou armature de déploiement radial (à la manière des implants transcutanés), qu'il s'agisse d'un stent autoexpansible en zigzags ou d'un stent déployable par un moyen de gonflage interne, tel qu'un ballon gonflable (comme dans US-A-5 195 984, par exemple).

Et, si comme le sont les manchons vasculaires chirurgicaux actuels, cette portion présente une capacité de déformation suivant l'axe du manchon, on pourra bénéficier d'un allongement important favorable à l'anastomose, la fixation vasculaire de la prothèse au vaisseau étant quant à elle assurée à l'autre extrémité par des crochets ou équivalents typiquement portés par l'armature ou stent.

En relation avec ce qui précède, on notera qu'en particulier, lorsqu'il s'agit d'une prothèse bifurquée (en "Y" inversé), la portion de manchon anastomosable s'étendra très avantageusement depuis l'extrémité de l'un au moins des tronçons secondaires (qui est aussi une extrémité libre de la prothèse), jusqu'au-delà de la zone de bifurcation des tronçons (voir figure 14 ci-après).

Concernant toujours la question du rapport de proportion en longueur entre la partie de manchon anastomosable et la partie supportée par le stent ou l'armature, on notera encore qu'en pratique, la longueur axiale de ce stent ou armature sera avantageusement juste suffisante pour assurer une ouverture radiale de la portion de manchon que cette armature ou ce stent supporte, ladite portion de manchon supportée ayant quant à elle avantageusement juste une longueur adaptée pour assurer l'étanchéité nécessaire vis-à-vis de la paroi du conduit considéré, de manière à éviter des fuites notables entre l'extrémité libre de cette portion de manchon et la paroi du conduit au contact de laquelle cette portion de manchon doit venir, sous la poussée de son armature support.

En d'autres termes, on réduira avantageusement au minimum la longueur du stent ou armature et de ladite portion correspondante de manchon supporté, pour réserver tout le reste de la longueur au manchon de type chirurgical anastomosable, optimisant ainsi les caractéristiques structurelles des deux parties de manchon eu égard à leur fonction, avec de bonnes garanties quant à l'ouverture de la portion de la prothèse "avec stent" et bonne étanchéité de contact à cet endroit, tout en obtenant des conditions favorables à l'anastomose, à l'extrémité libre opposée, et une étanchéité d'office sur l'essentiel de la longueur de l'implant (sans nécessiter de traitement annexe du manchon).

A noter encore qu'en relation avec ce qui précède, l'invention concerne également un ensemble comprenant l'implant déjà présenté et son matériel d'implantation par voie transcutanée endoluminale.

Dans ce qui suit, on va maintenant présenter une description plus détaillée de l'invention, à la fois dans sa constitution structurelle et dans le cadre de son procédé de mise en oeuvre.

Dans les dessins qui accompagnent la présente description :
- la figure 1 montre une prothèse bifurquée pour anévrisme,
- la figure 2 montre une prothèse tubulaire conforme à l'invention et destinée ici à servir d'allonge à l'un des tronçons de la prothèse de la figure 1,
- la figure 3 montre à petite échelle un guide-fil classique engagé à l'intérieur d'un dilatateur de voie d'abord percutanée, lui-même glissé dans une gaine d'implantation,
- la figure 4 montre à plus grande échelle un dispositif d'implantation pouvant être utilisé pour la mise en place de l'une et/ou l'autre des prothèses des figures 1 et 2,
- la figure 5 schématise une possibilité de mise en place par voie percutanée de la prothèse bifurquée de la figure 1,
- la figure 6 montre les deux prothèses des figures 1 et 2 en place dans des vaisseaux récepteurs,
- la figure 7 schématise la zone de l'intervention chirurgicale qui est plus précisément illustrée sur la figure 8,
- la figure 8 montre une étape de l'intervention chirurgicale où le praticien récupère la branche anastomosable de l'implant, après avoir réalisé le(s) sectionnement(s) nécessaire(s),
- la figure 9 montre en perspective un anneau utilisable comme moyen antihémorragie entre le vaisseau amont sectionné et la branche anastomosable de l'implant,
- les figures 10 et 11 figurent la mise en place de cet anneau, à l'endroit de la dénudation,
- la figure 12 montre les sutures entre ladite branche anastomosable de l'implant et la(les) zone (s) vasculaire(s) aval,
- les figures 13 et 14 montrent, de face, deux variantes de réalisation de l'implant (tube simple, figure 13 ; en "Y", figure 14), et
- la figure 15 est une vue locale en coupe selon le repère XV de la figure 13.

En relation avec ces figures, on ne va ci-après détailler que l'application de l'invention au traitement d'anévrismes iliaques primitifs débordant sur les artères hypogastrique et iliaque externe, même si éventuellement on pourrait envisager d'appliquer l'invention à d'autres conduits que des vaisseaux, ou du moins à d'autres affections vasculaires.

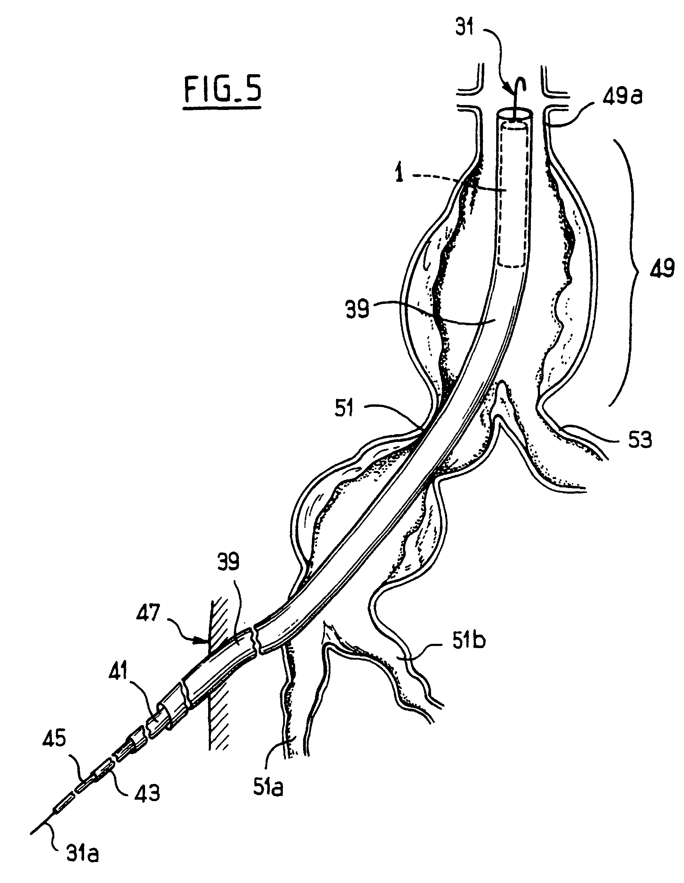

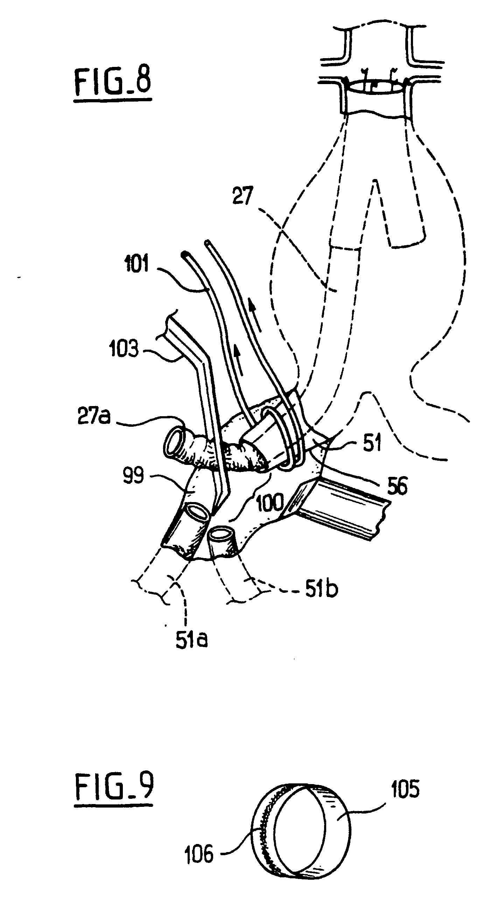

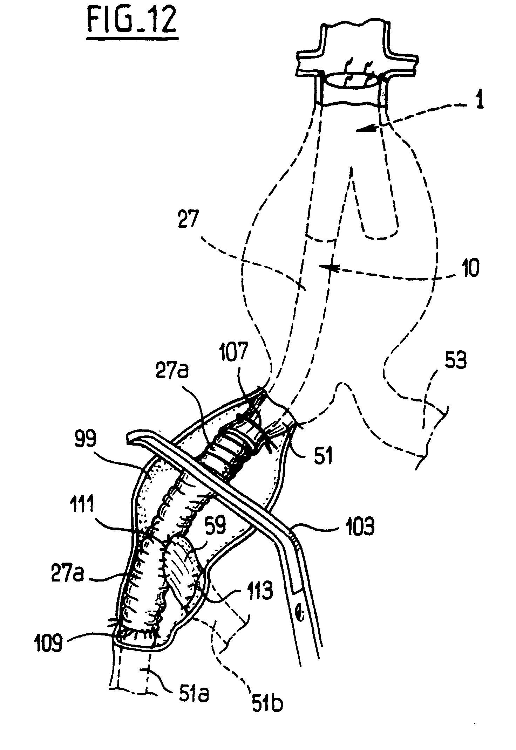

A noter également que les figures 5 et 6 montrent l'intérieur du corps du patient (en coupe), tandis que les figures 8, et 10 à 12 montrent (à l'exception de la partie supérieure des figures 8 et 12), en trait plein, la zone dénudée pour l'intervention chirurgicale (en 99) et, en pointillés, la situation de l'implant et l'anévrisme aortique.

Sur la figure 1 tout d'abord, est donc schématisée une prothèse vasculaire bifurquée 1.

La prothèse 1 comprend un tronçon tubulaire principal 3 se scindant en deux branches tubulaires 5, 7, toutes deux de longueur axiale plus courte que celle du tronçon 3.

Pour constituer cette forme, la prothèse est constituée d'un manchon extérieur en une matière souple 9 sans pratiquement de tenue mécanique intrinsèque, formant un tube en "Y" dessinant une sorte de culotte lorsque ce manchon est renforcé ou soutenu par une armature 11 à laquelle il est fixé.

L'armature intérieure 11 schématisée comprend un ou plusieurs fils métalliques (tels que de l'acier inoxydable) de quelques dixièmes de millimètre de diamètre (par exemple, de l'ordre de 0,1 mm à 0,5 mm) en zigzag enroulés en hélice d'axe 13 pour le tronçon principal 3, se scindant en deux hélices respectivement d'axes 15 et 17 et de diamètres légèrement inférieurs à celui de l'hélice du tronçon 3, pour les tronçons secondaires 5, 7.

Avantageusement, pour une bonne cohésion d'ensemble de l'armature 11, les apex (ou zones de courbure) 19 des zigzags de deux tours d'enroulement adjacents seront reliés entre eux par des attaches 21 pouvant consister en de petits anneaux, en des boucles nouées en fil de suture, voire en des points de soudure.

Pour plus de détail sur la réalisation de l'armature 11, on pourra se reporter, si nécessaire, à WO-A-95/21592.

On pourrait aussi utiliser un ou plusieurs stent(s) tubulaire(s) constitué(s) de plusieurs fils métalliques en zigzags.

L'armature 11 pourra ne s'étendre que sur une partie de la longueur de l'implant 1.

Pour assurer sa fixation au vaisseau et une liaison mécanique avec l'implant anastomosable 10 (zone 27, figure 2) ou 60 (stent 81, figure 13), l'implant 1 présentera un stent tubulaire ou annulaire au moins vers chacune de ses extrémités axiales opposées repérées par les zones 11a (extrémité proximale) et 11b, 11c (extrémité distale).

A noter que, quelle que soit la version retenue pour l'armature, sa constitution lui assurera de préférence une résistance à l'écrasement suivant l'axe général 13 et les axes de ramification 15 et 17. A noter également que cette armature pourra en outre être "autoexpansible" radialement, c'est-à-dire que les tronçons rectilignes de zigzag de ses fils d'armature auront tendance à s'écarter naturellement les uns des autres d'un angle α pouvant être compris entre environ 20° et 50° (diamètre d₁ ; figure 1).

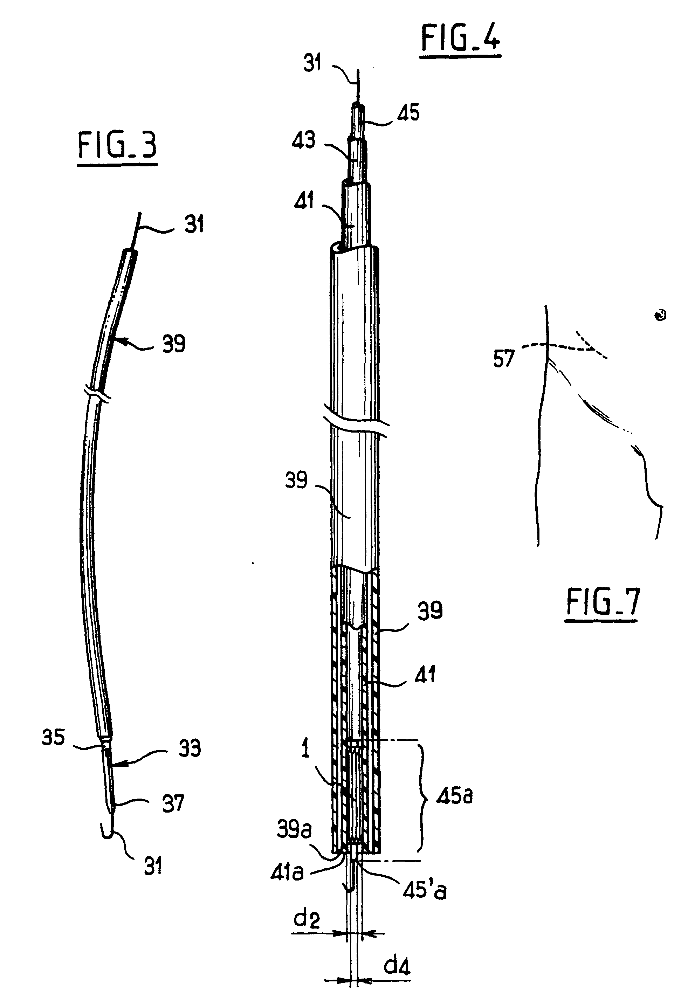

Bien entendu, une telle prothèse peut en outre être radialement comprimée pour être introduite par voie percutanée par une gaine ou un cathéter de petit diamètre, cet état "radialement resserré fermé" se matérialisant par une disposition sensiblement parallèle des tronçons rectilignes des fils de zigzag de l'armature (diamètre d₂ ; fig. 4), le tronçon de manchon fixé à ces fils suivant, quant à lui, le resserrement ou le déploiement radial de ladite armature.

A noter encore que des moyens (tels que des crochets) 23 de fixation de la prothèse au conduit considéré sont en outre de préférence prévus. Sur la figure 1, les crochets 23 sont soudés aux fils de zigzag situés à l'extrémité libre du tronçon principal 3, l'extrémité libre des jambes 5, 7, en étant dépourvue.

Sur la figure 2, il s'agit d'une prothèse 10 définissant un unique tube d'axe 25.

La prothèse 10 présente, à l'image de la prothèse 1, un manchon tubulaire d'habillage en tissu (ou équivalent) 27, soutenu intérieurement par une armature coaxiale 29.

L'armature 29 peut reprendre la structure filamentaire en zigzags de l'armature 11, dans la partie où elle se développe uniquement sur un tube (comme sur les tronçons 3, 5 ou 7). Sur la figure 2, on retrouve également les liens 21.

La particularité de la prothèse 10 consiste toutefois tout particulièrement en ce que son armature tubulaire 29 ne s'étend que sur une longueur axiale L₁ inférieure à la longueur totale L₂ du manchon tubulaire 27 qui l'entoure.

Ainsi, depuis son extrémité proximale 27', le manchon 27 n'est maintenu (par exemple intérieurement) par l'armature 29 que sur la longueur proximale L₁, sa partie terminale 27a (qui s'étend à sa suite jusqu'à son extrémité distale 27") en étant dépourvue. Si nécessaire, la portion terminale 27a pourrait être rapportée sur un tronçon principal de revêtement ayant la longueur L₁.

De ce qui précède, on aura compris qu'à l'image de la prothèse 1, la prothèse 10 peut être autoexpansible radialement (diamètre d₃ au repos ; figure 2) sous l'effet de son armature 29, le tronçon 27a du revêtement s'adaptant à l'état de la prothèse sur le reste de sa longueur.

Même si l'on peut envisager d'utiliser la prothèse 10 seule, l'exemple d'application ci-après prévoit que cette prothèse 10 soit une "prothèse de prolongement" de l'une des jambes 5, 7, de la prothèse bifurquée.

Sur les figures 3 à 5, on a représenté les moyens utilisés pour implanter par voie percutanée la prothèse 1 et/ou la prothèse 10.

Sur la figure 3, on voit une partie du matériel d'implantation comprenant un fin guide métallique 31 à extrémité distale recourbée sur lequel a été glissé un introducteur 33 à repère radio-opaque 35 et à bout effilé 37, une gaine introductrice 39 étant en outre glissée autour de l'introducteur 33.

Sur la figure 4, ont été schématisés les éléments qui vont être glissés sur le guide "J" 31, après retrait de l'introducteur 33. A l'intérieur de la gaine tubulaire 39, on trouve ainsi un cathéter intermédiaire 41 dans lequel sont logés concentriquement deux fins tubes de guidage destinés à faciliter la mise en place des prothèses.

A noter que sur la figure 4, les dimensions de la prothèse n'ont pas été respectées, celle-ci étant simplement schématisée dans son état radialement resserré, prête à être implantée. Concernant les tubes 43, 45, on notera que le tube intérieur 45 est plus long que le tube 43 et présente un diamètre d₄, ainsi qu'une longueur telle que sa partie terminale distale 45a traverse l'intérieur creux de la prothèse 1, pour se terminer en 45'a sensiblement au niveau de l'extrémité distale 39a de la gaine 39 à proximité immédiate de laquelle est également disposée la partie terminale distale 41a du cathéter 41 où a été préalablement préchargée la prothèse.

La procédure d'implantation peut être la suivante :

Supposons qu'une implantation par voie percutanée et approche fémorale par l'artère iliaque droite 51 a été retenue.

Après avoir ménagé une voie d'abord à travers la peau en 47 (voir figure 5), le guide-fil 31 est glissé par cette voie jusque dans l'aorte, de manière que son extrémité distale soit située un peu au-delà de l'extrémité distale 49a de l'anévrisme aortique 49. Sur le guide-fil, on glisse alors, depuis l'extérieur du corps du patient, l'introducteur 33 et la gaine 39.

Une fois cette gaine introduite jusqu'à proximité du bout distal du guide-fil, l'ensemble formé par le cathéter 41 renfermant la prothèse 1, ainsi que les deux tubes 43, 45, est poussé à l'intérieur de la gaine 39, jusqu'à ce que la prothèse parvienne à proximité de l'extrémité distale de cette gaine, comme illustré sur la figure 5. En maintenant à l'arrière la prothèse par appui contre le tube 43, et en tirant vers l'arrière la gaine 39 et le cathéter 41, la prothèse 1 est alors larguée dans l'aorte. Elle se déploit radialement jusqu'à venir se placer comme sur la figure

6, avec ses crochets 23 fixés au-delà de l'extrémité distale 49a de l'anévrisme et ses jambes 5, 7 dirigées vers les artères iliaques 51, 53. On notera, sur la figure 6, que la prothèse 1 a été implantée assez nettement à distance de la zone d'embranchement 54 des artères iliaques primitives, de telle sorte que les deux branches 5, 7 sont situées nettement, comme le tronçon 3, dans le conduit vasculaire "principal" (ici l'aorte 55).

Pour la pose de la prothèse 10, la gaine 39 a pu être laissée en place un peu plus bas dans l'aorte, le guide-fil 31 étant quant à lui de préférence maintenu à la même place.

On introduit alors à travers cette gaine repositionnée un deuxième cathéter intermédiaire dans la partie distale duquel on a préchargée la prothèse 10 et où ont également été disposés deux tubes de guidage identiques à ceux 43, 45, déjà présentés. A noter que la présence de la partie distale 45a du tube intérieur 45 au niveau de l'extrémité distale du cathéter 41 facilite l'engagement de cet ensemble sur l'extrémité proximale 31a du guide-fil 31. Guidé par ce guide-fil, qui traverse alors (dans l'exemple retenu) la branche 5 de la prothèse 1, l'ensemble précité va pouvoir être glissé jusqu'à l'intérieur de cette branche (les diamètres étant adaptés pour cela). Par un nouveau recul de la gaine 39 et surtout du cathéter intermédiaire combiné à un maintien arrière assuré par le tube 43, l'extrémité distale 10a de la prothèse 10 est placée à l'intérieur d'une partie de la jambe 5 où elle va s'ouvrir radialement, jusqu'à ce que du côté de l'extrémité 27' l'armature 29 vienne s'appuyer sur celle de la prothèse 1, dans la zone de stent 11b de celle-ci.

En poursuivant le retrait des différents tubes introducteurs le long du guide-fil, la prothèse 10 va se déployer progressivement jusqu'à l'intérieur de l'artère iliaque droite 51, de sorte que sa partie 27a constituée uniquement par le manchon 27 soit située à l'intérieur du débordement d'anévrisme iliaque repéré 56 sur la figure 6.

Il est rappelé qu'un débordement d'anévrisme de ce type est actuellement une contre-indication à un traitement par pose endoluminale percutanée de prothèse(s) vasculaire(s). Or, la nouvelle approche de l'invention qui associe à un tel traitement une intervention chirurgicale relativement légère, telle qu'en l'espèce une chirurgie de la bifurcation iliaque primitive par une voie d'abord iliaque limitée, permet d'utiliser une chirurgie beaucoup moins lourde que dans le cas d'un traitement entièrement chirurgical et donc de traiter des malades dans de meilleures conditions.

Une fois la "branche prothétique" 10 disposée comme sur la figure 6, l'intervention chirurgicale proprement dite va pouvoir débuter. Pour cela, le chirurgien incise le patient par voie iliaque, en zone sous péritonéale comme schématisé en 57 sur la figure 7.

Après avoir dénudé en 99 localement les vaisseaux 51, 51a, 51b et l'anévrisme 56 (qui s'étend au moins sur l'artère 51) et avoir pincé ou serré les zones qui doivent l'être (notamment via le lacet 101 et la pince 103 qui permettent temporairement de fermer au flux sanguin l'iliaque 51 et la branche prothétique 27 dans sa partie 27a), le chirurgien sectionne en 100 (figure 8) la zone vasculaire appropriée, en aval de l'anévrisme par référence au flux sanguin (en l'espèce le vaisseau iliaque 51, vers sa ramification en direction de 51a et 51b). Eventuellement, il coupe même le tronçon de 51 "malade".

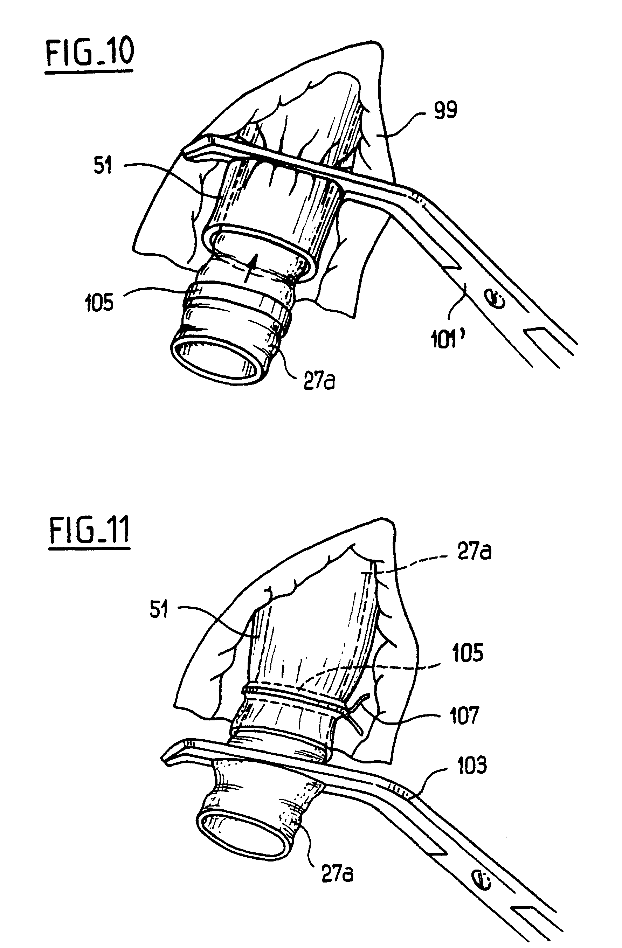

A travers la dénudation 99, le chirurgien récupère la branche prothétique anastomosable 27a qui passe à travers le tronçon vasculaire amont coupé 51. Le gaufrage de la branche prothétique permet une certaine adaptation en longueur (voir figure 10 où une pince 101' remplace le lacet 101).

Ensuite (voir figure 10), le chirurgien passe autour de la branche 27a (qui ressort) la bague d'appui 105 de la figure 9, laquelle présente une résistance à la compression. Il peut s'agir d'une bague continue (non fendue) en métal (acier inoxydable) ou en plastique bicompatible propre à enserrer ou à s'adapter étroitement autour de la branche 27a, avec par exemple une forme intérieure complémentaire (en 106) d'au moins un pli de gaufrage de cette branche pour favoriser l'étanchéité relative.

Autour de la bague 105 (correctement positionnée par glissement), le chirurgien place ensuite le tronçon vasculaire amont coupé 51 (figure 11) qu'il serre sur elle par l'intermédiaire du lien 107 sans écraser la branche 27a, grâce au moyen d'appui 105.

Il peut alors éventuellement déjà libérer le lacet 101 (ou la pince 101'), une hémorragie n'étant plus possible par fuite autour de la branche 27a de sang provenant de l'amont. La pince 103 est maintenue.

Le chirurgien relie ensuite (figure 12) au tronçon vasculaire sectionné aval 51a l'extrémité libre de ladite portion 27a, par anastomose (sutures 109), de manière à assurer une revascularisation appropriée. L'artère hypogastrique 51b peut ensuite être réimplantée, avec alors une suture latérale, soit directement sur la prothèse 27a, soit indirectement par un substitut de vaisseau 59 lui-même anastomosé à chaque extrémité (repères 111 et 113).

Une fois l'étanchéité assurée entre la branche prothétique 27a et la(les) zone(s) vasculaire(s) aval 51a (et 51b), le praticien retire la pince 103, laissant ainsi le sang passer librement depuis l'aorte, d'une part dans l'iliaque externe 51a, par l'intérieur de l'implant 1, 10, et d'autre part, dans l'artère hypogastique 51b, à travers le tronçon de substitution latéral 59, lui-même anastomosé à son extrémité aval à cette artère hypogastrique.

Par une seconde voie d'abord pratiquée pour accéder à l'artère iliaque gauche 53, une autre prothèse de prolongement "classique" (corespondant si nécessaire à la prothèse 10), peut alors être introduite, à nouveau de préférence par voie percutanée, jusqu'à l'intérieur de la seconde branche 7 de la prothèse bifurquée, de sorte à revasculariser correctement l'artère 53 (implantation non représentée).

A titre de variante de réalisation, on notera que les prothèses des figures 1 et 2 pourraient constituer un ensemble unique, l'une des jambes (5 ou 7), voire le tronçon 3, de la prothèse bifurquée 1 présentant alors une longueur beaucoup plus importante que l'(les) autre(s) et se terminant donc, dans sa partie distale, par le manchon tubulaire 27a dépourvu "d'armature".

A noter également que, si besoin était, la prothèse 10 pourrait même être utilisée seule (zone d'implantation non ramifiée).

Quoi qu'il en soit, une fois les opérations précitées effectuées, l'ensemble des moyens d'implantation de la(des) prothèse(s) est retiré du corps du patient (le matériel de mise en place percutanée ayant pu l'être avant le début de l'intervention en 99) et les voies d'abord intraluminale et chirurgicale pratiquées sont refermées.

Si l'on s'intéresse maintenant à la figure 13, l'implant anatomique illustré a été repéré 60.

Il s'agit, comme sur la figure 2, d'un implant qui se présente, au moins dans son état radialement déployé sur l'illustration, comme un tube unique de section sensiblement circulaire.

Sur sa longueur L₃, suivant son axe longitudinal 61, le diamètre peut être constant ou non.

A la différence de l'implant de la figure 2, celui de la figure 13 présente un tronçon anastomosable de manchon 63 d'une longueur L₄ égale à plus de la moitié de la longueur L₃ et, comme en l'espèce, à environ les 4/5 de cette longueur.

Sur cette longueur, la structure de la portion terminale distale 63 de manchon a intrinsèquement une certaine tenue et peut en particulier occuper naturellement, d'elle-même, la forme tubulaire souhaitée, sans nécessiter de moyens annexes. Au contraire, la portion d'extrémité proximale 65 (longueur L₅) ne présente la forme tubulaire ouverte de la figure 13 que sous l'effet de son armature ou stent de maintien 67 qui est ici dans son état radialement déployé et qui, à la manière des stents ou armatures déjà divulgués dans l'art antérieur, maintient le manchon 65 qui est fixé à lui par tout moyen approprié tel qu'un fil de suture, anneaux, oeillets, agrafes, ...

La structure du tronçon de manchon 65 sera très avantageusement celle d'un manchon de prothèse pour anévrisme à implantation endoluminale percutanée, à savoir une structure non autoportante, en matériau biocompatible adapté tel que polytétrafluoréthylène (PTFE), "Dacron", polyéthylène téréphtalate (PET), ou encore "Mylar", acétate de cellulose, matériau tissé ou tricoté, ...

Typiquement, ces matières ne sont pas travaillées pour assurer une autoportance.

Il s'agit classiquement d'une texture radialement extensible (élastiquement ou non) pour suivre la variation de diamètre de l'armature. Une autre solution peut être un filament non extensible, mais réalisé suivant un maillage déformable qui s'élargit lorsque l'implant est déployé.

Il peut même s'agir d'une structure intrinsèquement imparfaitement étanche au sang (sauf traitement ultérieur).

Au contraire, la structure de la portion 63 est sensiblement autoportante, à la manière des gaines de pontage en chirurgie vasculaire.

Il peut en particulier s'agir là d'une structure ayant une élasticité, ou du moins une capacité de déformation axiale importante, une étanchéité au sang et par contre pas ou peu de déformabilité radiale.

Du Dacron, du PTFE ou équivalent peut être utilisé. Une réalisation en polyester tricoté ou tissé comme dans US-A-3 986 828, avec une très faible porosité, est aussi envisageable.

Pour la déformabilité axiale, le tronçon anastomosable 63 peut être gaufré, comme l'illustrent les plis de gaufrage 69 des figures 13 et 14 (plis visibles également sur la figure 2).

Ainsi, sur un implant conforme à l'invention, le tronçon de manchon (27, 65) maintenu par son armature ou stent pourra avoir une première texture ou structure adaptée à la présence de cette armature lui assurant sa tenue mécanique et lui permettant de se déployer radialement une fois sortie du cathéter d'implantation, la portion complémentaire anastomosable du manchon pouvant être par contre optimisée pour favoriser la reprise chirurgicale, une fois la prothèse implantée et étant donc pour cela particulièrement appropriée pour être suturée, tout en étant intrinsèquement sensiblement étanche au sang, naturellement radialement ouverte pour autoriser d'office une circulation interne de liquide (section circulaire en particulier) et offrant plus de résistance à la déformation radiale que l'autre portion, (cette seconde portion étant au surplus avantageusement non extensible radialement).

Comme montré figure 15, l'épaisseur "intrinsèque" e₁ des portions 63, 65 pourra être identique. L'épaisseur totale e₂ de 63 pourra être par contre supérieure, du fait du gaufrage. L'épaisseur e₁ sera par exemple de 0,15 mm à 0,5 mm, environ. L'épaisseur e₂ pourra atteindre 0,5 mm à 1mm, environ.

Comme déjà indiqué, les tronçons 63 et 65 pourront non seulement être intrinsèquement de structure différente, mais également être réalisés en deux parties distinctes reliées entre elles par tout moyen approprié, tel qu'un fil de suture, agrafes, oeillets, ..., ceci sensiblement coaxialement et bord à bord, voire avec un léger chevauchement, en favorisant l'étanchéité relative entre les deux tronçons vis-à-vis du liquide corporel devant y circuler.

Sur la figure 13, on remarquera également que l'armature 67 se présente comme un anneau tubulaire constitué par un fil métallique (ou plusieurs fils bout à bout) conformé(s) en zigzags ou en méandres fermés sur eux-mêmes et enroulés ainsi sur un seul étage ou un seul tour 31, avec comme particularité que le tronçon anastomosable 63 et cette armature 67 ne se chevauchent pas ou pratiquement pas l'un l'autre, de telle sorte que l'armature peut jouer son plein effet vis-à-vis du tronçon de manchon "non autoportant" 65, en particulier au moment où il doit être radialement déployé pour venir s'appliquer étroitement contre la paroi du conduit porteur.

Toutes les remarques qui viennent d'être faites au regard de la figure 13, s'appliquent également à l'implant 70 en "Y" inversé de la figure 14, qui est bien entendu implantable par voie endoluminale percutanée.

En plus de ce qui précède, cet implant insérable dans un vaisseau comprend une jambe courte 71 et une jambe longue 73 réalisées dans un matériau anastomosable, en l'espèce gaufré, ce manchon en "Y" remontant sur le tronçon principal 75 de l'implant, jusqu'au-delà de l'embranchement 77, sur environ les 6/7 de la longueur L₆ du manchon dans son ensemble.

En partie haute du tronçon principal 75, on retrouve à nouveau une texture de manchon radialement déformable, en 79, dont la tenue mécanique est assurée par un stent intérieur 81 fixé à ce tronçon.

En l'espèce, il s'agit d'un stent "en croisillons", en métal déployé, par exemple en fil à mémoire de forme, tel qu'en "Nitinol" (marque déposée).

Ce stent passe d'un diamètre resserré propre à l'implantation de la prothèse par voie percutanée endoluminale à un diamètre élargi dans un état implanté à l'intérieur du conduit récepteur, sous l'effet d'un ballon de gonflage autour duquel la prothèse sera montée dans son matériel d'introduction, à la manière de ce qui est par exemple décrit dans EP-A-684 022.

Sur la figure 13 comme sur la figure 14, des moyens de fixation de l'extrémité proximale "avec stent" de la prothèse, tels que des crochets 83, sont prévus.

Le reste de l'implant (63 ou 71, 73, 75) est par contre dépourvu de moyens d'accrochage et de stent, puisque la destination de cette portion est d'être anastomosée à, ou vers, son extrémité libre, telle que 63a, 71a ou 73a (qui est aussi l'extrémité libre distale de la prothèse ou de la jambe considérée).

Dans ce qui précède, on a dit que la portion "anastomosable" (27a, 63, 71, 73) de l'implant est dépourvue de stent de soutien. Ceci n'empêche pas l'utilisation d'un manchon anastomosable (par exemple en PTFE) doté d'une.spirale (pouvant être dans la même matière) ou plus généralement d'une structure radialement résistante, favorisant la résistance à la plicature et pouvant assurer une légère élasticité longitudinale (possibilité alors d'utiliser un manchon non gaufré). Une telle structure "radialement résistante" n'a, quoi qu'il en soit, pas la fonction d'un "stent".

## Revendications

1. Prothèse vasculaire (10, 60, 70) adaptée pour être implantée par voie endoluminale percutanée, comprenant un manchon souple (27, 65, 79) ayant une portion terminale vasculairement anastomosable de manchon (27a, 63, 71, 73) qui prolonge, ou est rapportée sur, ledit manchon souple, au-delà de l'armature, cette portion étant donc adaptée pour être anastomosée, en particulier par suture, à au moins un vaisseau ou substitut de vaisseau, et une armature ou stent tubulaire (29, 67, 81) à laquelle ou auquel est lié essentiellement coaxialement le manchon, l'armature étant propre à occuper un premier diamètre ou un deuxième diamètre supérieur au premier, pour que la prothèse se présente comme un tube unique (10, 60) ou bifurqué (1, 70) occupant un état radialement resserré pour son implantation vasculaire percutanée ou un état radialement déployé, une fois vasculairement implanté, **caractérisée en ce qu'**elle comprend en outre
- un moyen annulaire d'appui (105) à disposer autour de la portion terminale anastomosable de manchon (27a ; 63 ; 71, 73, 75) pour obtenir une étanchéité vis-à-vis du sang entre ledit moyen annulaire et cette portion terminale,
- et un moyen de fixation étanche (107) pour fixer ainsi, autour de ce moyen d'appui, le tronçon de vaisseau coupé (51) à travers lequel est passée cette portion terminale anastomosable.

2. Prothèse selon la revendication 1, **caractérisée en ce que** la prothèse est une prothèse bifurquée (70) pour un traitement d'anévrisme, comprenant un tronçon tubulaire principal (75) raccordé à des tronçons tubulaires secondaires (71, 73) dont l'un se termine à son extrémité libre par ladite portion terminale vasculairement anastomosable de manchon.

3. Prothèse selon la revendication 2, **caractérisée en ce que** l'extrémité libre du tronçon tubulaire principal (79) est pourvue de crochets (23, 83) de fixation vasculaire, tandis que l'extrémité libre des tronçons tubulaires secondaires (71, 73) en est dépourvue.

4. Prothèse vasculaire selon l'une quelconque des revendications précédentes, **caractérisée en ce que** :
- la partie du manchon située essentiellement en regard de l'armature (29, 67; 81) est radialement extensible et/ou n'occupe sa forme tubulaire radialement ouverte que sous l'action de ladite armature qui la maintient et présente alors son deuxième diamètre,
- la portion terminale anastomosable de manchon (27a, 63, 71, 73, 75) est moins extensible radialement que l'autre portion (27, 65, 79, 27) et/ou occupe naturellement d'elle-même une forme tubulaire radialement ouverte.

5. Prothèse selon la revendication 4, **caractérisée en ce que** les matières ou textures de constitution et/ou les épaisseurs (e₁, e₂) desdites parties de manchon respectivement en regard de l'armature et anastomosable sont différentes.

6. Prothèse vasculaire selon l'une des revendications 4 et 5, **caractérisée en ce que** le tronçon anastomosable de manchon (63, 71, 73, 75) présente une capacité de déformation suivant l'axe du tube.

7. Prothèse selon l'une quelconque des revendications 4 à 6, **caractérisée en ce que** le tronçon anastomosable de manchon ne s'étend pas, ou pratiquement pas, en regard de l'armature (67, 81, 11, 29).

8. Prothèse selon l'une quelconque des revendications précédentes, **caractérisée en ce que** le tronçon anastomosable de manchon (63 ; 71, 73, 75) s'étend sur au moins l'essentiel de la longueur totale (L₃, L₆) du manchon.

9. Ensemble comprenant :
- la prothèse vasculaire à anastomoser selon l'une quelconque des revendications 1 à 8, laquelle se présente comme un tube unique, non bifurqué (10),
- une autre prothèse vasculaire (1) de traitement d'anévrisme, adaptée pour être implantée vasculairement par voie endoluminale percutanée, et se présentant comme un tube bifurqué,
- et un matériel d'implantation par voie endoluminale percutanée desdites prothèses (1, 10, 60, 70).

10. Ensemble selon la revendication 9 comprenant :
- une seconde prothèse vasculaire (1) de traitement d'anévrisme, cette seconde prothèse, qui se présente comme un tube bifurqué, comprenant elle-même un manchon (9) pour canaliser du sang et au moins un stent ou une armature tubulaire (11) pour son manchon, ce stent, ou cette armature étant présent (en 11a ; 11b, 11c) au moins aux deux extrémités axiales de la prothèse (1) et occupant un premier diamètre ou un deuxième diamètre supérieur au premier, de manière que cette seconde prothèse soit adaptée pour être implantée par voie percutanée endoluminale,
- les deux prothèses (1, 10 ; 60, 70) étant en outre adaptées pour une liaison entre ladite seconde prothèse vasculaire bifurquée (1, 70) et l'extrémité de ladite première prothèse opposée à celle pourvue dudit tronçon terminal à anastomoser.

## Claims

1. Vascular prosthesis (10, 60, 70) arranged to be implanted by the percutaneous endoluminal route, comprising a flexible sleeve (27, 65, 79) comprising a vascularly anastomosable terminal portion of sleeve (27a, 63, 71, 73) which extends, or is added onto, the said flexible sleeve, beyond the armature, this portion configured to be anastomosed, in particular by suture, to at least one vessel or vessel substitute, and a tubular armature or stent (29, 67, 81) to which the sleeve is connected essentially coaxially, the armature being capable of having a first diameter or a second diameter larger than the first, so that the prosthesis is in the form of a single tube (10, 60) or bifurcated tube (1, 70) assuming a radially constricted state for its percutaneous vascular implantation, or a radially opened out state, once it is vascularly implanted, **characterized in that** it additionally comprises:
an annular support means (105) configured to be arranged around the anastomosable terminal portion of sleeve (27a; 63 ; 71, 73, 75) in order to obtain a leaktight seal with respect to the blood between the said annular means and the terminal portion,
and a leaktight fixing means (107) in order thus to fix, round the support means, the cut vessel section (51) through which the anastomosable terminal portion is passed.

2. Prosthesis according to claim 1, **characterized in that** the prosthesis is a bifurcated prosthesis (70) for treatment of an aneurism, comprising a principal tubular section (75) connected to secondary tubular sections (71, 73) one of which ends at its free end in the said vascularly anastomosable terminal portion of sleeve.

3. Prosthesis according to claim 2, **characterized in that** the free end of the principal tubular section (79) is provided with vascular fixing hooks (23, 83), while the free end of the secondary tubular sections (71, 73) is devoid of them.

4. Vascular prosthesis according to anyone of preceding claims, **characterized in that**:
the part of the sleeve located essentially facing the armature (29, 67, 81) is radially extensible and/or assumes its radially open tubular form only under the action of the said armature which supports it, and then exhibits its second diameter,
the anastomosable terminal portion of sleeve (27a, 63, 71, 73, 75) is less radially extensible than the other portion (27, 65, 79, 27) and/or naturally assumes by itself a radially open tubular form.

5. Prosthesis according to claim 4, **characterized in that** the constituent materials or textures and/or the thicknesses (e₁, e₂) of the said sleeve parts, respectively facing the armature and anastomosable, are different.

6. Vascular prosthesis according to anyone of claims 4 and 5, **characterized in that** the anastomosable sleeve section (63, 71, 73, 75) has a capacity for deformation along the axis of the tube.

7. Prosthesis according to according to anyone of claims 4 to 6, **characterized in that** the anastomosable sleeve section does not extend, or practically does not extend, facing the armature (67, 81, 11, 29).

8. Prosthesis according to anyone of preceding claims, **characterized in that** the anastomosable sleeve section (63; 71, 73, 75) extends over at least the greater part of the total length (L₃, L₆) of the sleeve.

9. Assembly comprising:
the vascular prosthesis to be anastomosed according to anyone of claims 1 to 8, which is in the form of a single, non-bifurcated tube (10),
another vascular prosthesis (1) for treatment of an aneurism, configured to be implanted vascularly by the percutaneous endoluminal route, and being in the form of a bifurcated tube,
and equipment for implantation of the said prostheses (1, 10, 60, 70) by the percutaneous endoluminal route.

10. Assembly according to claim 9, including:
a second vascular prosthesis (1) for treatment of an aneurism, the second prosthesis, which is in the form of a bifurcated tube, itself comprising a sleeve (9) for channelling the blood and at least one tubular stent or armature (11) for its sleeve, the stent or armature being present (at 11a; 11b, 11c) at least at the two axial ends of the prosthesis (1) and having a first diameter or a second diameter larger than the first, in such a way that the second prosthesis is arranged to be implanted by the endoluminal percutaneous route,
the said two prostheses (1, 10; 60, 70) configured to be arranged for connection between the said bifurcated second vascular prosthesis (1, 70) and the opposite end of the said first prosthesis from that provided with the said terminal section to be anastomosed.

## Patentansprüche

1. Gefäßprothese (10, 60, 70), die geeignet ist, auf endoluminalem, perkutanem Weg implantiert zu werden, wobei sie eine elastische Muffe (27, 65, 79) mit einem vaskulär zur Anastomose fähigen Endteil der Muffe (27a, 63, 71, 73) hat, der die flexible Muffe über der Armierung verlängert oder auf ihr angestückt ist, wobei dieser Teil folglich insbesondere durch Naht an mindestens einem Gefäß oder Gefäßersati zur Anastomose geeignet ist, und eine röhrenförmige Armierung oder einen Stent (29, 67, 81) hat, mit welcher oder mit welchem die Muffe im wesentlichen koaxial verbunden ist, wobei die Armierung geeignet ist, einen ersten Durchmesser oder einen zweiten Durchmesser, der größer ist als der erste, einzunehmen, damit sich die Prothese als ein einziges (10, 60) oder gegabeltes (1, 70) Rohr darstellt, das für seine perkutane Gefäßimplantation einen radial eingeengten Zustand oder einen radial entfalteten Zustand einnimmt, wenn es einmal vaskulär implantiert ist, **dadurch gekennzeichnet, daß** sie femer aufweist:
- ein ringförmiges Stützmittel (105) zur Anordnung um den zur Anastomose fähigen Endteil der Muffe (27a; 63; 71, 73, 75), um eine Dichtigkeit gegenüber dem Blut zwischen dem ringförmigen Mittel und diesem Endteil zu erhalten,
- und ein Mittel zur dichten Befestigung (107), um so um dieses Stützmittel herum den geschnittenen Gefäßabschnitt (51) zu befestigen, durch welchen dieser zur Anastomose fähige Endteil durchgeführt wird.

2. Prothese nach Anspruch 1, **dadurch gekennzeichnet, daß** die Prothese eine gegabelte Prothese (70) für eine Aneurisma-Behandlung ist, wobei sie einen röhrenförmigen Hauptabschnitt (75) aufweist, der mit röhrenförmigen Nebenabschnitten (71, 73) verbunden ist, von denen einer an seinem freien Ende durch den vaskulär zur Anastomose fähigen Endteil der Muffe endet.

3. Prothese nach Anspruch 2, **dadurch gekennzeichnet, daß** das freie Ende des röhrenförmigen Hauptabschnittes (79) Haken (23, 83) zur vaskulären Befestigung hat, während das freie Ende der röhrenförmigen Nebenabschnitte (71, 73) diese nicht hat.

4. Gefäßprothese nach einem der vorhergehenden Ansprüche, **dadurch gekennzeichnet, daß**:
- der Teil der Muffe, der im wesentlichen gegenüber der Armierung (29, 67, 81) liegt, radial ausdehnbar ist und/oder seine röhrenförmige, radial geöffnete Form nur unter Tätigkeit der Armierung einnimmt, die ihn hält, und dann seinen zweiten Durchmesser aufweist,
- der zur Anastomose fähige Endteil der Muffe (27a, 63, 71, 73, 75) radial weniger ausdehnbar ist als der andere Teil (27, 65, 79, 27) und/oder natürlich von selbst eine röhrenförmige, radial geöffnete Form einnimmt.

5. Prothese nach Anspruch 4, **dadurch gekennzeichnet, daß** die Materialien oder Beschaffenheitsstrukturen und/oder die Dicken (e₁, e₂) der Teile der Muffe jeweils gegenüber der Armierung und der Fähigkeit zur Anastomose unterschiedlich sind.

6. Gefäßprothese nach einem der Ansprüche 4 und 5, **dadurch gekennzeichnet, daß** der zur Anastomose fähige Abschnitt der Muffe (63, 71, 73, 75) eine Fähigkeit zur Verformung entlang der Rohrachse aufweist.

7. Prothese nach einem der Ansprüche 4 bis 6, **dadurch gekennzeichnet, daß** der zur Anastomose fähige Abschnitt der Muffe sich nicht oder praktisch nicht gegenüber der Armierung (67, 61, 11, 29) erstreckt.

8. Prothese nach einem der vorhergehenden Ansprüche, **dadurch gekennzeichnet, daß** sich der zur Anastomose fähige Abschnitt der Muffe (63; 71, 73, 75) auf mindestens dem Hauptteil der Gesamtlänge (L₃, L₆) der Muffe erstreckt.

9. Aufbau mit:
- der Gefäßprothese zur Anastomose nach einem der Ansprüche 1 bis 8, welche sich als ein einziges, nicht gegabeltes Rohr (10) darstellt,
- einer anderen Gefäßprothese (1) zur Aneurisma-Behandlung, die geeignet ist, vaskulär auf perkutanem, endoluminalem Weg implantiert zu werden, und die sich als ein gegabettes Rohr darstellt,
- einem Material zur Implantation auf perkutanem, endoluminalem Weg der Prothesen (1, 10, 60, 70).

10. Aufbau nach Anspruch 9 mit:
- einer zweiten Gefäßprothese (1) zur Aneurisma-Behandlung, wobei diese zweite Prothese, die sich als ein gegabeltes Rohr darstellt, selbst eine Muffe (9) aufweist, um Blut zu kanalisieren und mindestens einen Stent oder eine röhrenförmige Armierung (11) für seine Muffe, wobei der Stent oder diese Armierung bei (11a; 11b, 11c) an mindestens zwei axialen Enden der Prothese (1) vorhanden ist und einen ersten Durchmesser oder einen zweiten Durchmesser, der größer ist als der erste, derart einnimmt, daß diese zweite Prothese geeignet ist, auf perkutanem, endoluminalem Weg implantiert zu werden,
- wobei die zwei Prothesen (1, 10; 60, 70) ferner für eine Verbindung zwischen der zweiten gegabelten Gefäßprothese (1, 70) und dem Ende der ersten Prothese geeignet sind, das dem ohne Endabschnitt zur Anastomose gegenüber liegt.
